# EUROPEAN PATENT APPLICATION

(11) **EP 3 181 041 A1**
(43) Date of publication of application: **21.06.2017**
(21) Application number: 15201274.6
(22) Date of filing: 18.12.2015
(51) Int. Cl.: A61B 5/0402, A61B 5/048, A61B 5/00

(54) **SYSTEM AND METHOD FOR IN EAR EEG SENSING AND AUDIO DELIVERY FOR SLEEP ENHANCEMENT**

(71) Applicant: Koninklijke Philips N.V., 5656 AE Eindhoven (NL)
(72) Inventor: The designation of the inventor has not yet been filed
(74) Representative: de Haan, Poul Erik

(57) **Abstract**

The present disclosure pertains to a system configured to manage a sleep session of a subject. In some embodiments, the system comprises an ear insert that includes one or more sensory stimulators and one or more electroencephalogram (EEG) sensors. Traditionally, electrodes used during EEG measurements are either applied to directly to a person's head or integrated into some kind of cap to be worn by the subject of the EEG measurements. Applying EEG electrodes to a person's head or wearing a cap with electrodes is cumbersome and obtrusive. In addition, sensory stimulation is usually provided with separate stimulation equipment that is separate from the electrodes applied to the subject's head or the cap worn by the subject, or are at best separately coupled to the cap worn by the subject. This further increases the cumbersome nature of EEG measurement for a given subject, especially during sleep.

## Description

### FIELD OF THE INVENTION

The present disclosure pertains to a system configured to manage a sleep session of a subject.

### BACKGROUND OF THE INVENTION

Sensory stimulation during sleep is known. Sensory stimulation during sleep is often applied continuously and/or at intervals that do not correspond to sleeping patterns of a subject. Sensory stimulation is often provided by devices such as speakers or lights located near the subject. Sensory stimulation is typically controlled based on information from electrodes attached to a subject with stickers or via a headband or hood worn by the subject.

### SUMMARY OF THE INVENTION

Accordingly, one or more aspects of the present disclosure relate to a system configured to manage a sleep session of a subject. While management of sleep is used throughout this disclosure as an example, this is not intended to be limiting. The system described herein may be used anytime accessing brain signals and/or providing sensory stimulation in an unobtrusive manner is advantageous. As additional examples, the present system may be used while a subject is awake or asleep to monitor alertness, for neurofeedback training, to test mental vitality, for home health monitoring, for wearable personal audio based treatments, and/or for other purposes. The system comprises an ear insert, one or more hardware processors, and/or other components. The ear insert is configured to engage an ear of the subject. The ear insert comprises one or more sensory stimulators configured to provide sensory stimulation to the subject during the sleep session and one or more sensors configured to generate output signals conveying information related to brain activity of the subject. The one or more hardware processors are configured by machine-readable instructions to: determine one or more brain activity parameters of the subject based on the output signals; and control the one or more sensory stimulators based on the one or more brain activity parameters to provide sensory stimulation to the subject to enhance and/or otherwise adjust brain activity (e.g., slow wave sleep) in the subject during the sleep session.

Another aspect of the present disclosure relates to a method for managing a sleep session of a subject with a sleep management system. The sleep management system comprises one or more hardware processors and an ear insert that includes one or more sensory stimulators and one or more sensors. The method comprises: engaging, with the ear insert, an ear of the subject; providing, with the one or more sensory stimulators, sensory stimulation to the subject during the sleep session; generating, with the one or more sensors, output signals conveying information related to brain activity of the subject; determining, with the one or more hardware processors, one or more brain activity parameters of the subject based on the output signals; and controlling, with the one or more hardware processors, the one or more sensory stimulators based on the one or more brain activity parameters to provide sensory stimulation to the subject to enhance and/or otherwise adjust brain activity (e.g., slow wave sleep) in the subject during the sleep session.

Still another aspect of the present disclosure relates to a system for managing a sleep session of a subject. The system comprises: means for engaging an ear of the subject; wherein the means for engaging comprises means for providing sensory stimulation to the subject during the sleep session; and means for generating, with the one or more sensors, output signals conveying information related to brain activity of the subject. The system further comprising means for determining one or more brain activity parameters of the subject based on the output signals; and means for controlling the one or more sensory stimulators based on the one or more brain activity parameters to provide sensory stimulation to the subject to enhance and/or otherwise adjust brain activity (e.g., slow wave sleep) in the subject during the sleep session.

In some embodiments, cardiac "artifacts" in the output signals may be used by the system as additional information on which to base the sensory stimulation to influence sleep and/or other activities. For example, this may include enhancing slow wave sleep, facilitating falling asleep with neurofeedback, causing the subject to wake up, limiting a nap of the subject, and/or other activities.

These and other objects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the disclosure.

### BRIEF DESCRIPTION OF THE DRAWINGS

FIG. 1 is a schematic illustration of a system configured to manage a sleep session of a subject.
FIG. 2 illustrates interchangeable earbud attachments configured to removably couple with a base of an ear insert of the system.
FIG. 3 illustrates a second embodiment of an earbud attachment configured to removably couple with a base of the ear insert.
FIG. 4 illustrates forming ear inserts based on a wax model of the ear canal of the subject.
FIG. 5 illustrates custom forming the ear insert based on three dimensional data representative of the ear of the subject.
FIG. 6 illustrates an electronic model used when custom forming the ear insert.
FIG. 7 illustrates a segment of data from an overnight EEG recording using the system.
FIG. 8 illustrates two possible embodiments of an electronic circuit formed by a transmitter included in the ear insert.
FIG. 9 illustrates a method for managing a sleep session of a subject with a management system.

### DETAILED DESCRIPTION OF THE EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

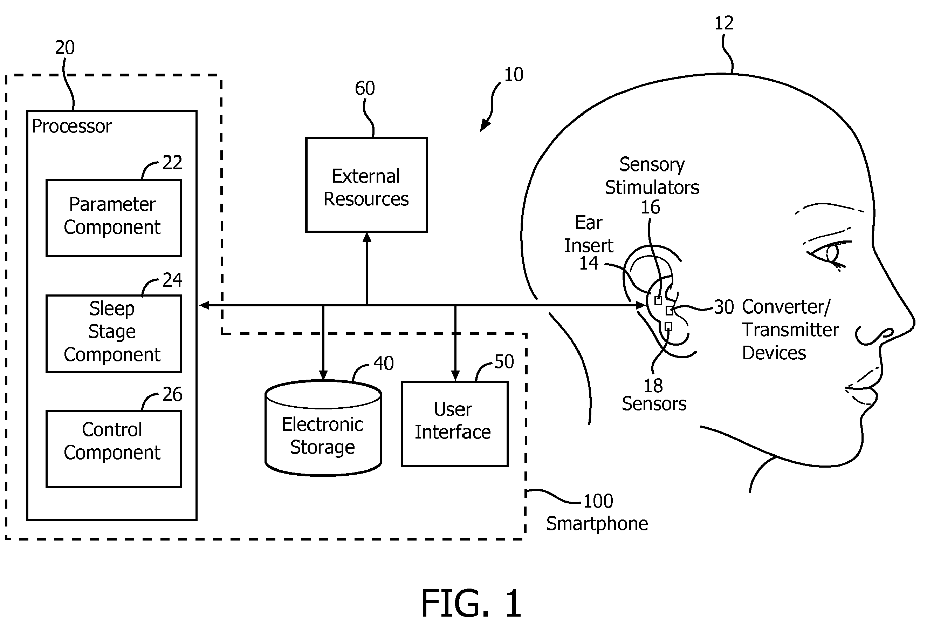

FIG. 1 is a schematic illustration of a system 10 configured to manage a sleep session of a subject 12. In some embodiments, system 10 comprises one or more of an ear insert 14, a processor 20, electronic storage 40, a user interface 50, external resources 60, and/or other components. Ear insert 14 includes one or more sensory stimulators 16, one or more sensors 18, and/or other components. Traditionally, electrodes used during electroencephalogram (EEG) measurements are either applied to directly to a person's head or integrated into some kind of cap to be worn by the subject of the EEG measurements. Applying EEG electrodes to a person's head or wearing a cap with electrodes is cumbersome and obtrusive, especially during sleep when EEG measurements are commonly gathered. In addition, when sensory stimulation is provided to a subject, the sensory stimulation is provided with separate stimulation equipment (e.g., speakers, lights) that is separate from the electrodes applied to the subject's head or the cap worn by the subject, or are at best separately coupled to the cap worn by the subject. This further increases the cumbersome nature of EEG measurement for a given subject, especially during sleep. EEG measurements are being used more and more in consumer products and small less obtrusive EEG measurement devices are desired in such consumer products. System 10 provides a small unobtrusive system that facilitates comfortable provision of sensory stimulation and gathering of EEG measurements for subject 12.

While EEG measurement during sleep is used throughout this disclosure as an example, this is not intended to be limiting. System 10 may be used anytime accessing brain signals and/or providing sensory stimulation in an unobtrusive manner is advantageous. As additional examples, system 10 may be used while a subject is awake or asleep to monitor alertness, for neurofeedback training, to test mental vitality, for home health monitoring, for wearable personal audio based treatments, and/or for other purposes.

Ear insert 14 is configured to engage an ear of subject 12. Engaging an ear of subject 12 includes being removably inserted into a portion of the ear canal of subject 12, being removably inserted into a portion of the outer ear of subject 12, begin removably coupled (e.g., hooked, clipped, clamped, etc.) with an outer portion of the ear of subject 12, and/or other engagement. In some embodiments, one or more portions of ear insert 14 that touch the ear of subject 12 are formed from conductive rubber, a conductive silver textile, a silicone material that is described in US patent application 62/167384 which is hereby incorporated by reference in its entirety, and/or other materials. In some embodiments, these conductive materials form a portion sensor 18 (described below).

In some embodiments, ear insert 14 comprises an earbud manufactured with one or more generic and/or standard shapes configured to couple with a range of user ear sizes and/or dimensions. In some embodiments, ear insert 14 comprises interchangeable earbud attachments in a range of sizes configured to removably couple with a base of ear insert 14 such that subject 12 may find an interchangeable earbud attachment that is most comfortable and attach it to the base. In some embodiments, ear insert 14 is custom formed for the ear of subject 12. Ear insert 14 is custom formed based on three dimensional data representative of the ear of subject 12, a physical mold/model of the ear of subject 12, and/or other information. The three dimensional data, the mold/model, and/or other information representative of the ear of subject 12 is generated via one or more devices included in external resources 60 (described below) and/or via other devices. For example, external resources 60 may include three dimensional scanning equipment (e.g., cameras, processors, display devices, software applications, and/or other equipment), mold making devices and/or materials (e.g., wax and/or other materials), and/or other external resources configured to generate the three dimensional data, the mold/model, and/or the other information representative of the ear of subject 12.

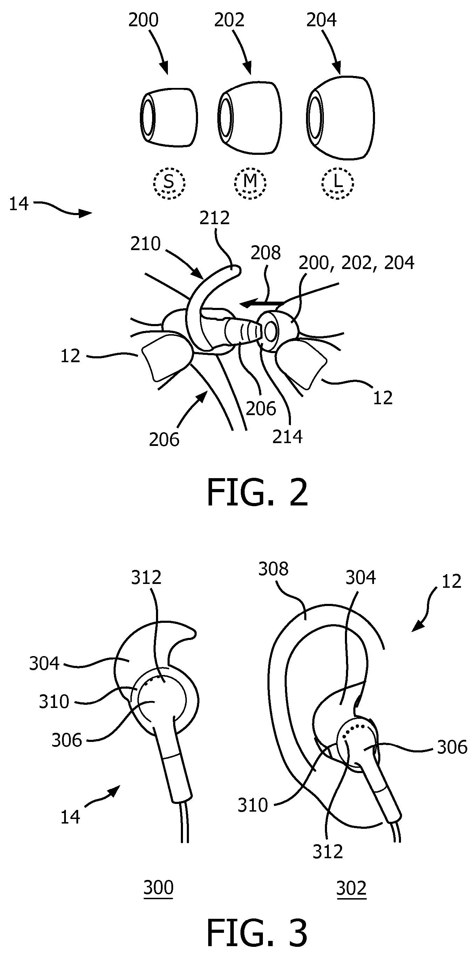

FIG. 2-6 illustrate various embodiments of ear inserts 14. FIG. 2 illustrates interchangeable earbud attachments 200, 202, 204 in a range of sizes (e.g., small (S), medium (M), and large (L) configured to removably couple 208 with a base 206 of ear insert 14 such that subject 12 may find an interchangeable earbud attachment that is most comfortable and attach it to the base. In the embodiment shown in FIG. 2, earbud attachments 200, 202, 204 are formed from the conductive materials described above such that earbud attachments 200, 202, 204 form a portion of sensor 18 (shown in FIG. 1). Base 206 includes electronic components that form a portion of sensor 18, sensory stimulator 16, and/or other components of system 10. Base 206 and attachments 200, 202, 204 form correspond clamping surfaces 214 configured to removably couple with each other. Clamping surface 214 on base 206 includes a conductive surface 218 such that electrical signals passing through earbud attachments 200, 202, 204 are received by base 206. In some embodiments, base 206 may include one or more features 210 to enhance coupling with the ear of subject 12. FIG. 2 illustrates, for example, features 210 comprising a hook 212 that wraps around the ear of subject 12.

FIG. 3 illustrates two views 300, 302 of a second embodiment of an earbud attachment 304 configured to removably couple with a base 306 of ear insert 14. Earbud attachment 304 is configured to removably couple with base 306 via a portion 310 of earbud attachment 304 that wraps around a portion 312 of base 306. Earbud attachment 304 is removably inserted into a portion of the outer ear 308 of subject 12. In the embodiment shown in FIG. 3, earbud attachment 304 may be formed from the conductive materials described above such that electrical signals passing through earbud attachment 304 are received by base 306.

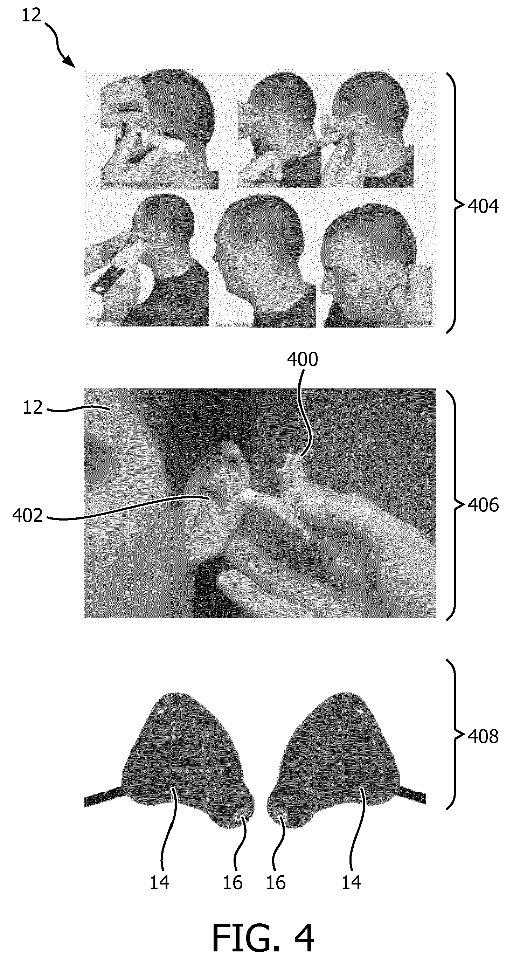

FIG. 4 illustrates forming ear insert(s) 14 based on a wax model 400 of the ear canal 402 of subject 12. FIG. 4 illustrates sensory stimulators 16 formed integrally with ear inserts 14. At an operation 404, wax is inserted into ear canal 402 of subject 12 and allowed to harden/cool. At an operation 406, wax model 400 is removed from ear canal 402. At an operation 408, ear inserts 14 are formed based on wax model 400. Forming ear inserts 14 based on wax model 400 may include using wax model 400 as a mold positive to form a mold for ear inserts 14, electronically scanning and/or otherwise imaging wax model 400 and generating ear inserts 14 based on electronic dimensional data generated during the scan, and/or other formation of ear inserts 14 based on wax model 400. Ear inserts 14 are formed from the conductive materials described above such that sensors 18 are formed integrally with ear inserts 14.

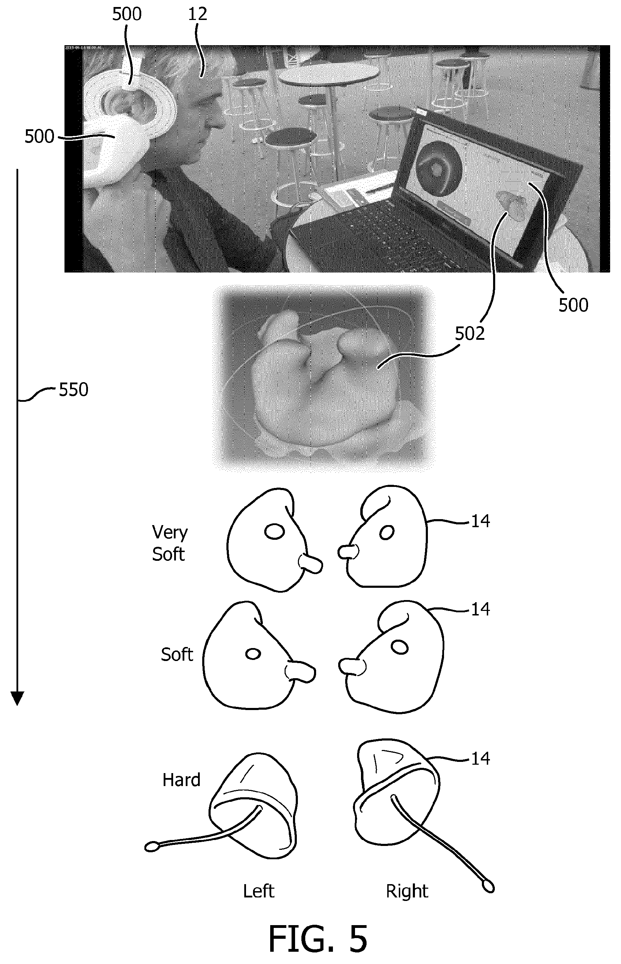

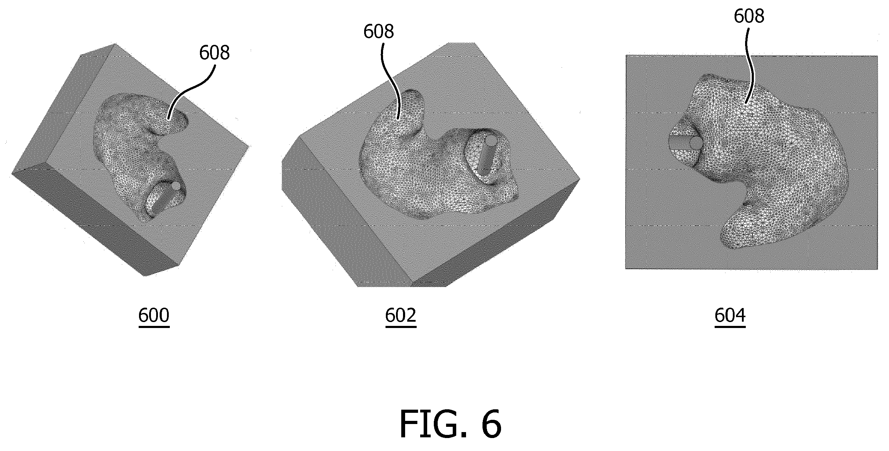

FIG. 5 and 6 illustrate custom forming ear insert 14 (shown in FIG. 5) based on three dimensional data representative of the ear of subject 12 (FIG. 5). For example, FIG. 5 illustrates a process 550 comprising generating three dimensional data representative of the ear of subject 12 by scanning the inner ear of subject 12 with scanning equipment 500 (e.g., included in external resources 60 shown in FIG. 1), generating a CAD (for example) model 502 of the inner ear based on the data obtained via scanning equipment 500, and forming ear inserts 14 based on the CAD file. FIG. 5 illustrates multiple ear inserts 14 formed based on the CAD file. As shown in FIG. 5, corresponding left and right ear inserts 14 are made at three different levels of material elasticity (e.g., very soft, soft, and hard).

In some embodiments, an ear insert 14 maybe fabricated directly based a CAD file and/or other electronic files (e.g., the electronic file is transmitted to a machine that cuts, prints, solidifies and/or otherwise forms the insert). In some embodiments, the CAD and/or other electronic files are used to model a mold and/or generate other components used to fabricate an ear insert 14. For example, FIG. 6 illustrates views 600, 602, 604 of a model 608 of a mold used to generate an ear insert 14. Model 608 is generated (e.g., using a modeling program such as Solidworks) based on three dimensional data generated during scans like the one illustrated in FIG. 5 and/or generated during other scans. Whether a custom ear insert 14 is fabricated directly based on the three dimensional data or fabricated from a mold or other components formed based on the three dimensional data, ear insert 14 may be customized based on the three dimensional data to fill the ear canal of subject 12 and much of the outer ear of subject 12 without protruding from the head of subject 12 so as to enable comfort during sleep and/or other activities (e.g., maximizing the surface area ear insert 14 that is in contact with the ear of subject 12 without hindering comfort). In some embodiments, ear insert 14 may be customized to include a small canal built into ear insert 14 (e.g., with an approximate diameter of about 2-3 mm) to facilitate hearing ambient sounds (e.g., during sleep and/or while awake). In some embodiments, ear insert 14 may be customized such than an area of ear insert 14 under the tragus of subject 12 is smoothed to allow the tragus room for lying against the earpiece should the subject 12 elect to sleep on their side, for example.

In should be noted that any description herein of a single ear insert 14 is not intended to be limiting. Ear insert 14 may include two separate ear inserts 14 (e.g., one for each ear of subject 12) having substantially similar components and/or functionality as described herein.

Returning to FIG. 1, as described above, ear insert 14 includes one or more sensory stimulators 16, one or more sensors 18, and/or other components. One or more sensory stimulators 16 and one or more sensors 18 are formed integrally with ear insert 14 such that ear insert 14, one or more sensory stimulators 16, and one or more sensors 18 appear to form a single unified physical object that is comfortable for subject 12 to insert and remove from his or her ears.

Sensory stimulator 16 is configured to provide sensory stimulation to subject 12 during a sleep session. In some embodiments, sensory stimulator 16 may be configured to induce and/or adjust slow wave activity (SWA) in subject 12 through non-invasive brain stimulation and/or other methods. Sensory stimulator 16 may be configured to induce and/or adjust SWA through non-invasive brain stimulation using sensory stimuli. The sensory stimuli include sounds, vibrations, and/or other stimuli. For example, sensory stimulator 16 may be configured to induce and/or adjust SWA via audio stimulation (e.g., tones, music, and/or other auditory stimulation), vibratory stimulation, and/or other sensory stimulation of subject 12. Examples of sensory stimulator 16 may include one or more of a music player, a tone generator, a unit to deliver vibratory stimulation (also known as somato-sensory stimulation), and/or other devices.

Sensor 18 is configured to generate output signals conveying information related to brain activity of subject 12. Information related to brain activity of subject 12 may include and/or be indicative of slow wave activity (SWA) in subject 12 (e.g., slow waves, sleep spindles, K-complexes, etc.) sleep pressure in subject 12, a sleep stage of subject 12, and/or other characteristics of subject 12. Sensor 18 may comprise one or more sensors that generate output signals conveying such information directly. For example, sensor 18 may include electrodes configured to detect electrical activity resulting from current flows within the brain of subject 12. Sensor 18 may comprise one or more sensors that generate output signals conveying information related to brain activity of subject 12 indirectly. For example, one or more sensors 18 may generate an output based on a heart rate of subject 12 (e.g., sensor 18 may be a heart rate sensor), movement of subject 12 (e.g., sensor 18 may include an accelerometer such that sleep may be analyzed using actigraphy signals), respiration of subject 12, and/or other characteristics of subject 12. In some embodiments, a portion of sensor 18 is formed by the conductive material described above that contacts the ear of subject 12.

In some embodiments, sensor 18 comprises one or more EEG electrodes and/or other sensors configured to generate EEG and/or other output signals that convey the information related to the brain activity of subject 12. The one or more EEG electrodes may comprise a reference electrode, a sensing electrode, and/or other electrodes. SWA corresponds to the power of an EEG signal in the 0.5-4.5 Hz band. SWA has a typical behavior throughout cyclic variations of a given sleep session. SWA increases during non-rapid eye movement sleep (NREM), declines before the onset of rapid-eye-movement (REM) sleep, and remains low during REM. SWA in successive NREM episodes progressively decreases from one episode to the next. SWA may be estimated from an electroencephalogram (EEG) for subject 12 during a given sleep session. SWA is related to sleep pressure. Sleep pressure may be thought of as a need for sleep in subject 12. The higher the SWA is, the higher the need for sleep is. The decrease in SWA throughout a sleep episode reflects the dissipation of sleep need. Sleep pressure dissipates and/or decreases as subject 12 sleeps. The dissipation dynamics depend on the given subject. The manner in which dissipation occurs regulates the length of a given sleep session and is linked to the temporal dynamics of SWA. The current sleep stage of subject 12 may correspond to one or more of non-rapid eye movement (NREM) stage N1, stage N2, or stage N3 sleep, and/or rapid eye movement (REM) sleep. In some embodiments, NREM stage 3 or stage 2 sleep may be slow wave sleep.

Although sensor 18 is illustrated at a single location in communication with an ear of subject 12, this is not intended to be limiting. Sensor 18 may include sensors disposed in a plurality of locations, such as for example, within (or in communication with) both ears of subject 12, and/or in other locations. In embodiments where ear insert 14 comprises two separate ear inserts for the ears of subject 12 and sensor 18 includes one or more EEG electrodes, reference and/or sensing electrodes may be included in one or both of the ear inserts 14 in the ears of subject 12. For example, an ear insert 14 in a left ear of subject 12 may include a reference electrode and an ear insert 14 in a right ear of subject 12 may include a sensing electrode (but no reference electrode). As another example, ear inserts 14 in both ears of subject 12 may include both a reference electrode and a sensing electrode. As a third example, an ear insert 14 in one ear of subject 12 may include a reference electrode and an ear insert 14 in the other ear of subject 12 may include multiple sensing electrodes.

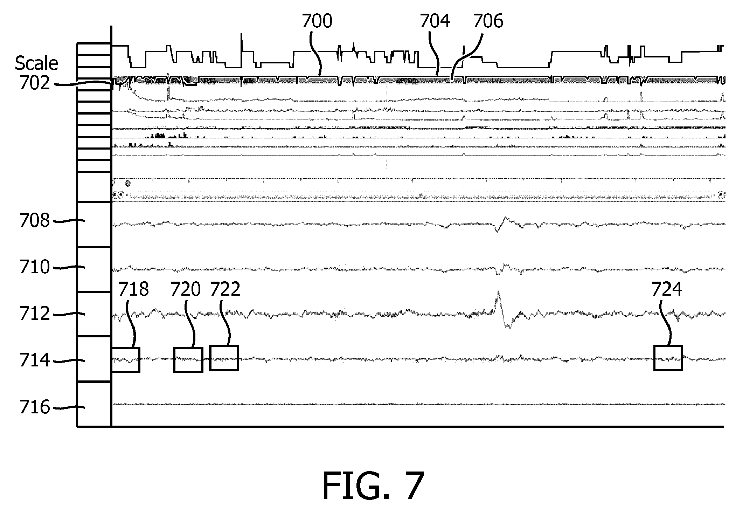

By way of a non-limiting example, FIG. 7 illustrates a thirty second segment of data from an overnight EEG recording using system 10 (FIG. 1). Signal strength from EEG electrodes (e.g., sensors 18 shown in FIG. 1) in ear inserts 14 positioned in the left and right ears of a subject is shown by line 700 on scale 702. The signal strength is at 100% when line 700 is at the top 704 of scale 702 and at 0% when line 700 is at the bottom 706 of scale 702. As shown in FIG. 7, the signal strength from the EEG electrodes (sensors 18) in ear inserts 14 is typically 100% with only minor interruptions during the 30 second period. Brain activity during the 30 second period is indicated by lines 708-716. At locations 718, 720, 722, and 724, line 714 indicates presence of sleep spindles and/or K-complexes.

Returning to FIG. 1, in some embodiments, ear insert 14 includes converter and transmitter devices 30. Converter devices 30 are configured to convert, if necessary, the output signals, and/or the information in the output signals from sensor 18 for transmission to, and receipt by processor 20. Transmitter devices 30 are configured to transmit and receive signals. In some embodiments, the signals may be transmitted and/or received wirelessly and/or via wires. In some embodiments, transmitter devices 30 are configured to transmit and receive the signals via wires, one or more radio channels of a radio link, via a cellular communication network, via Wi-Fi, via Bluetooth, and/or via other communication methods. In some embodiments, transmitter devices 30 are configured to transmit and receive communication signals substantially simultaneously.

In some embodiments, converter devices 30 are configured to transform the output signals into one or more signals suitable for a microphone input of a smartphone (described below), a USB input of a smartphone, and/or other signals for analysis by processor 20. In some embodiments, converter devices 30 comprise an electronic circuit comprising instrumentation amplifier and gain devices, a signal conditioner comprising a high and low pass filter, an ADC converter, and/or other components. By way of a non-limiting example, conversion can be done close to the electrodes in the ear but also an analog signal can be transmitted to another location (e.g. a cabled module on the neck, chin, or arm of subject 12) where the analog signal is converted into a digital signal.

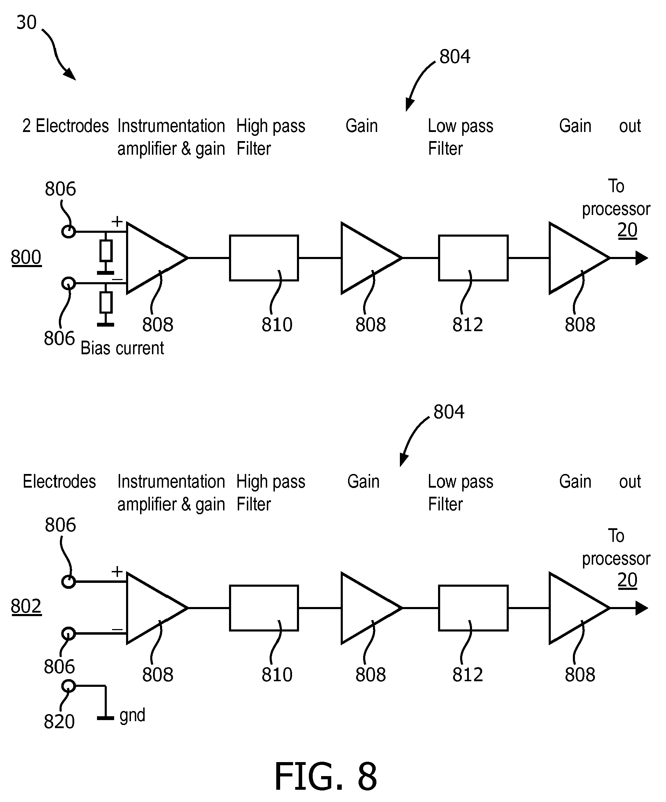

Two possible embodiments 800, 802 (and there are many more) of the electronic circuit 804 formed by converter and/or transmitter devices 30 (shown in FIG. 1) are illustrated in FIG. 8. As shown in FIG. 8, embodiment 800 includes two electrodes 806 (sensors 18 shown in FIG. 1), amplifier and gain devices 808, a high pass filter 810, and a low pass filter 812. Embodiment 802 illustrates, alternatively, a third electrode 820 may be used as a grounding/reference electrode. Third electrode 820 may be positioned outside the ear of subject 12 (FIG. 1) and/or may be integrated into one or both ear inserts 14 (FIG. 1) in the ears of subject 12. In embodiments with third electrode 820 integrated into an ear insert 14, ear insert 14 comprises two isolated electrode areas, one for third electrode 820 and the other for electrodes 806. In some embodiments, additional electrodes (not shown) may be located on the forehead via, for example, a strap from the left to right ears including electrodes touching the forehead. Similarly, a chin strap and/or a neck strap may be envisioned.

Returning to FIG. 1, processor 20 is configured to provide information processing capabilities in system 10. As such, processor 20 may comprise one or more of a digital processor, an analog processor, and a digital circuit designed to process information, an analog circuit designed to process information, a state machine, and/or other mechanisms for electronically processing information. Although processor 20 is shown in FIG. 1 as a single entity, this is for illustrative purposes only. In some embodiments, processor 20 may comprise a plurality of processing units. These processing units may be physically located within the same device (e.g., within a smartphone associated with subject 12), or processor 20 may represent processing functionality of a plurality of devices operating in coordination.

As shown in FIG. 1, processor 20 is configured to execute, by machine-readable instructions, one or more computer program components. The one or more computer program components may comprise one or more of a parameter component 22, a sleep stage component 24, a control component 26, and/or other components. Processor 20 may be configured to execute components 22, 24, and/or 26 by software; hardware; firmware; some combination of software, hardware, and/or firmware; and/or other mechanisms for configuring processing capabilities on processor 20.

It should be appreciated that although components 22, 24, and 26 are illustrated in FIG. 1 as being co-located within a single processing unit, in embodiments in which processor 20 comprises multiple processing units, one or more of components 22, 24, and/or 26 may be located remotely from the other components. The description of the functionality provided by the different components 22, 24, and/or 26 described below is for illustrative purposes, and is not intended to be limiting, as any of components 22, 24, and/or 26 may provide more or less functionality than is described. For example, one or more of components 22, 24, and/or 26 may be eliminated, and some or all of its functionality may be provided by other components 22, 24, and/or 26. As another example, processor 20 may be configured to execute one or more additional components that may perform some or all of the functionality attributed below to one of components 22, 24, and/or 26.

Parameter component 22 is configured to determine one or more brain activity parameters of subject 12 and/or other parameters. Parameter component 22 is configured to determine the brain activity parameters of subject 12 based on the output signals from sensors 18 and/or other information. In some embodiments, parameter component 22 receives signals from transmitter devices 30. In some embodiments, parameter component 22 is configured to convert the signals from transmitter devices 30 back into EEG signals for parameter determination and/or analysis. In some embodiments, one or more of the brain activity parameters determined by parameter component 22 include parameters related to and/or indicative of SWA (e.g., the power in the slow wave activity band (0.5-4.5Hz) or other frequency bands), sleep pressure, and/or a sleep stage of subject 12 during the sleep session, an effectiveness of the sensory stimulation (e.g., the effectiveness is related to an increase in SWA caused by the provided sensory stimuli), arousals of subject 12, evoked potentials, and/or other parameters.

In some embodiments, parameter component 22 identifies SWA in subject 12 based on an analysis of the information conveyed by the output signals of sensors 18. The analysis may include generating and/or monitoring an EEG during a sleep session of subject 12. In some embodiments, the analysis may include detecting slow wave sleep based on a power in a delta band and/or a power in a beta band of the EEG. The power in the delta band is usually defined as the power in the frequency range 0.5-4.5 Hz although there is no standard definition of the frequency limits. The power in the beta band is usually defined as the power in the frequency range 15-30Hz although variations in limits of the range are very common. In some embodiments, the analysis may include detecting presence and/or determining a density/rate of sleep spindles based on the EEG power in the spindle band (e.g., about 11 to 15 Hz), K-complexes, individual slow waves, and/or other information. Parameter component 22 may determine sleep pressure based on the SWA. In some embodiments, parameter component 22 may be configured to generate a hypnogram based on the determined parameters. The EEG and/or the hypnogram may be displayed, for example, by user interface 50 (described below).

Sleep stage component 24 is configured to identify sleep stages (e.g., N1, N2, N3) while subject 12 is sleeping. Sleep stage component 24 is configured to identify sleep stages based on the output signals from sensor 18, information from parameter component 22, and/or other information. For example, sleep stage component may identify sleep stages based on the SWA in subject 12, based on the hypnogram generated by parameter component 22, and/or other information. Sleep stages may include rapid eye movement (REM) sleep, and/or non-rapid eye movement (NREM) stage N1, stage N2, and/or stage N3 sleep. In some embodiments, stage N3 sleep may be and/or correspond to slow wave sleep. In some embodiments, stage N2 and/or stage N3 sleep may be slow wave sleep. In some embodiments, slow waves may not be present throughout the whole N3 period, for example, but it may be significantly more likely that such slow waves are present during N3. Slow waves may also be present (although to a lesser extent) during N2, for example.

Control component 26 is configured to control sensory stimulator 16 (e.g., by sending signals received by transmitter devices 30) to provide sensory stimulation to subject 12. Control component 26 is configured to control sensory stimulator 16 based on the one or more brain activity parameters, the sleep stage of subject 12, and/or other information. Control component 26 is configured to control sensory stimulator 16 to provide sensory stimulation to subject 12 to enhance slow wave sleep in subject 12 during the sleep session and/or for other reasons.

In some embodiments, controlling sensory stimulator 16 to enhance slow wave sleep in subject 12 includes controlling sensory stimulator 16 to increase and/or decrease SWA in subject 12 during sleep. In some embodiments, control component 26 is configured to determine a timing for delivery of the sensory stimulation. Control component 26 is configured to determine the timing for delivery of the sensory stimulation based on the information from parameter component 22, information from sleep stage component 24, information from sensors 18, and/or other information. The timing for delivery of sensory stimulation may correspond to sleep stages associated with slow wave activity (e.g., N2, N3), may be determined based on (filtered) real-time output signals, and/or may have other timings. For example, control component 26 may be configured to determine a timing for delivery of sensory stimulation such that the determined timing corresponds to sleep stages N2 and N3 while subject 12 is sleeping. Control component 26 may be configured to determine a timing for delivery of sensory stimulation such that the determined timing corresponds to sleep stages associated with slow wave activity because the likelihood for slow-wave induction, and/or adjustment during a specific sleep stage may be comparatively higher than in other sleep stages, subject 12 may be less likely to be awakened by the sensory stimuli, and/or for other reasons. Control component 26 is configured to control sensory stimulator 16 to provide sensory stimulation to subject 12 based on the determined timing and/or other information.

In some embodiments, control component 26 is configured to control sensory stimulator 16 to provide the sensory stimuli in the form of auditory tones that are brief in duration (e.g., about 50ms long), have a predetermined frequency, have a predetermined intensity, are separated from each other by an inter-tone-interval and/or have other characteristics. In some embodiments, one or more of the auditory tone duration, the predetermined frequency, the predetermined intensity, the inter-tone-interval, and/or other characteristics of the sensory stimuli may be programmed at manufacture, set by a user via user interface 50, determined by system 10 based on previous sleep sessions of subject 12, determined based on the current sleep session, and/or determined by other methods.

In some embodiments, processor 20 is included in a smartphone 100 associated with subject 12 and/or other computing devices. In some embodiments, processor 20 is included in a tablet computer, a laptop computer, a desktop computer, a server computer, and/or other computing devices. Smartphone 100 comprises an input device (not shown in FIG. 1) configured to receive the information in the output signals generated by sensor 18 and/or other information. The input device may be and/or include a microphone included in the smartphone, a USB input device, an Apple Lightning type connector (which can also provide power), a combined microphone/earphones jack, and/or other devices. In some embodiments, as described above, converter devices 30 are configured to convert the output signals, and/or the information in the output signals from sensor 18 for transmission to, and receipt by, the smartphone input device. In some embodiments, processor 20 is configured such that parameter component 22, sleep stage component 24, control component 26, and/or other components of processor 20 form an electronic application (an "app") running on processor 20. In some embodiments, the app (as described above related to parameter component 22, sleep stage component 24, and control component 26) reads the transformed signal from converter and transmitter devices 30, converts it back to an EEG signal (if necessary), and analyzes the signal to determine the information described above.

Electronic storage 40 comprises electronic storage media that electronically stores information. The electronic storage media of electronic storage 40 may comprise one or both of system storage that is provided integrally (i.e., substantially non-removable) with system 10 and/or removable storage that is removably connectable to system 10 via, for example, a port (e.g., a USB port, a firewire port, etc.) or a drive (e.g., a disk drive, etc.). Electronic storage 40 may comprise one or more of optically readable storage media (e.g., optical disks, etc.), magnetically readable storage media (e.g., magnetic tape, magnetic hard drive, floppy drive, etc.), electrical charge-based storage media (e.g., EPROM, RAM, etc.), solid-state storage media (e.g., flash drive, etc.), and/or other electronically readable storage media. Electronic storage 40 may store software algorithms, information determined by processor 20, information received from subject 12, and/or other information that enables system 10 to function as described herein. Electronic storage 40 may be (in whole or in part) a separate component within system 10, or electronic storage 40 may be provided (in whole or in part) integrally with one or more other components of system 10 (e.g., with processor 20 in smartphone 100 associated with subject 12).

User interface 50 is configured to provide an interface between system 10 and subject 12 and/or other users through which subject 12 and/or other users may provide information to and receive information from system 10. This enables data, cues, results, and/or instructions, and any other communicable items, collectively referred to as "information," to be communicated between a user (e.g., subject 12) and one or more of sensory stimulator 16, sensor 18, processor 20, electronic storage 40, external resources 60, and/or other components of system 10. For example, an EEG may be displayed to subject 12 and/or a caregiver via user interface 50.

Examples of interface devices suitable for inclusion in user interface 50 comprise a keypad, buttons, switches, a keyboard, knobs, levers, a display screen, a touch screen, speakers, a microphone, an indicator light, an audible alarm, a printer, a tactile feedback device, and/or other interface devices. In some embodiments, user interface 50 comprises a plurality of separate interfaces. In some embodiments, user interface 50 comprises at least one interface that is provided integrally with processor 20 (e.g., included in smartphone 100 associated with the user), ear insert 14, and/or other components of system 10. As an example, in some embodiments, user interface 50 comprises an auditory user interface such that subject 12 can give auditory feedback signals via tones or speech. As another example, user interface 50 may be configured such that touching; tapping; mouth, jaw, and/or facial motions; and/or other gestures or motions with various parts of the body can be inputs to control the device.

It is to be understood that other communication techniques, either hard-wired or wireless, are also contemplated by the present disclosure as user interface 50. For example, the present disclosure contemplates that user interface 50 may be integrated with a removable storage interface provided by electronic storage 40. In this example, information may be loaded into system 10 from removable storage (e.g., a smart card, a flash drive, a removable disk, etc.) that enables the user(s) to customize the implementation of system 10. Other exemplary input devices and techniques adapted for use with system 10 as user interface 50 comprise, but are not limited to, an RS-232 port, RF link, an IR link, modem (telephone, cable or other). In short, any technique for communicating information with system 10 is contemplated by the present disclosure as user interface 50.

External resources 60 may include sources of information (e.g., databases, websites, etc. storing three dimensional data representative of the ear of subject 12), equipment configured to obtain, analyze, and/or generate a three dimensional representation of the ear of subject 12, one or more servers outside of system 10, a network (e.g., the internet), physical ear mold/model generation equipment, equipment related to Wi-Fi technology, equipment related to Bluetooth® technology (e.g., to facilitate communication between ear insert 14 and processor 20 for example), data entry devices, sensors, scanners, cameras, and/or other resources. In some embodiments, some or all of the functionality attributed herein to external resources 60 may be provided by resources included in system 10. External resources 60 may be configured to communicate with sensory stimulator 16, sensor 18, converter and transmitter devices 30, processor 20, electronic storage 40, user interface 50, and/or other components of system 10 via wired and/or wireless connections, via a network (e.g., a local area network and/or the internet), via cellular technology, via Wi-Fi technology, and/or via other resources.

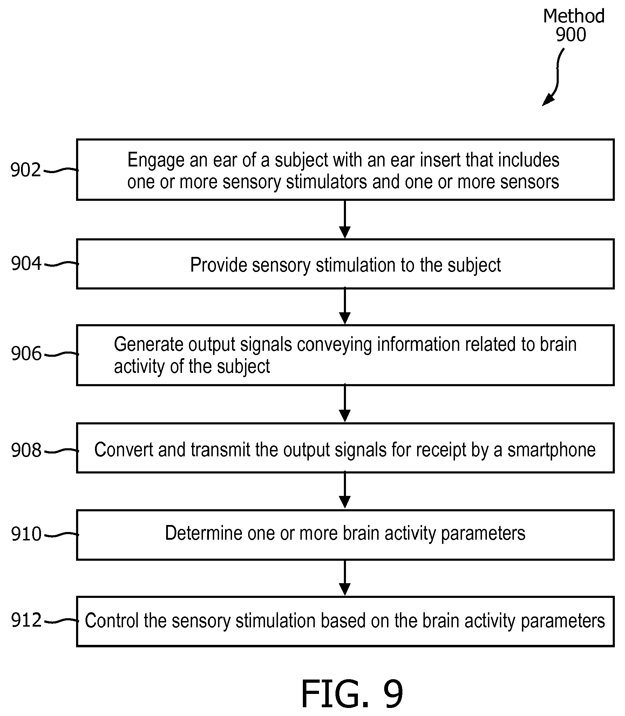

FIG. 9 illustrates a method 900 for managing a sleep session of a subject with a management system. The system comprises one or more hardware processors, an ear insert that includes one or more sensory stimulators, one or more sensors, and a transmitter, and/or other components. The one or more hardware processors are configured, by machine-readable instructions, to execute one or more computer program components. The computer program components include one or more of a parameter component, a control component, and/or other components. The one or more hardware processors and/or the computer program components may be included in a smartphone associated with the subject and/or other computing devices. The operations of method 900 presented below are intended to be illustrative. In some embodiments, method 900 may be accomplished with one or more additional operations not described, and/or without one or more of the operations discussed. Additionally, the order in which the operations of method 900 are illustrated in FIG. 9 and described below is not intended to be limiting.

In some embodiments, method 900 may be implemented in one or more processing devices (e.g., a digital processor, an analog processor, a digital circuit designed to process information, an analog circuit designed to process information, a state machine, and/or other mechanisms for electronically processing information). The one or more processing devices may include one or more devices executing some or all of the operations of method 900 in response to instructions stored electronically on an electronic storage medium. The one or more processing devices may include one or more devices configured through hardware, firmware, and/or software to be specifically designed for execution of one or more of the operations of method 900.

At an operation 902, an ear of a subject is engaged with the ear insert. Engaging the ear of the subject may include removably coupling with a portion of the ear canal of the subject, a portion of the outer ear/earlobe of the subject, and/or other coupling. The ear insert includes the one or more sensory stimulators and the one or more sensors. The ear insert may engage the ear of the subject such that the one more sensory stimulators are positioned to deliver sensory stimulation to the subject and the one or more sensors are positioned to generate output signals conveying information related to the brain activity of the subject. In some embodiments, operation 902 includes custom forming the ear insert for the subject based on three dimensional data representative of the ear of the subject. In some embodiments, operation 902 is performed by an ear insert that is the same as or similar to ear insert 14 (shown in FIG. 1 and described herein).

At an operation 904, sensory stimulation is provided to the subject. In some embodiments, the sensory stimulation includes auditory tones and/or other sensory stimulation. In some embodiments, operation 904 is performed by one or more sensory stimulators similar to and/or the same as sensory stimulator(s) 16 (shown in FIG. 1 and described herein). It should be noted that, in some embodiments, operation 904 is optional. In some cases sensory stimulation may be used to trigger evoked potentials measured and analyzed to gain insights on alertness and/or fatigue, for example. For other kinds of stimulation and/or feedback based on brain activity monitoring, sensory stimulation up front is not necessary.

At an operation 906, output signals conveying information related to brain activity of the subject are generated. In some embodiments, the output signals are EEG output signals that convey the information related to the brain activity of the subject. In some embodiments, the one or more sensors comprise one or more EEG electrodes configured to generate EEG output signals that convey the information related to the brain activity of the subject. In some embodiments, the one or more EEG electrodes comprise a reference electrode, a sensing electrode, and/or other electrodes. In some embodiments, operation 906 is performed by sensors the same as or similar to sensors 18 (shown in FIG. 1 and described herein).

At an operation 908, the output signals are converted (if necessary) and transmitted for receipt by a smartphone. In some embodiments, the output signals are converted and transmitted for receipt by a smartphone input device. In some embodiments, the input device includes a microphone, a USB input device, and/or other input devices. In some embodiments, operation 908 is performed by a transmitter included in the ear insert that is the same as or similar to converter and transmitter devices 30 (shown in FIG. 1 and described herein).

At an operation 910, one or more brain activity parameters are determined. In some embodiments, operation 910 is performed by a processor component that is the same as or similar to parameter component 22 (shown in FIG. 1 and described herein).

At an operation 912, the sensory stimulation is controlled based on the brain activity parameters. In some embodiments, operation 912 includes controlling, with the one or more processors, the one or more sensory stimulators based on the one or more brain activity parameters to provide sensory stimulation to the subject to enhance slow wave sleep in the subject during the sleep session. In some embodiments, operation 912 is performed by a processor component that is the same as or similar to control component 26 (shown in FIG. 1 and described herein).

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the description provided above provides detail for the purpose of illustration based on what is currently considered to be the most practical and preferred embodiments, it is to be understood that such detail is solely for that purpose and that the disclosure is not limited to the expressly disclosed embodiments, but, on the contrary, is intended to cover modifications and equivalent arrangements that are within the spirit and scope of the appended claims. For example, it is to be understood that the present disclosure contemplates that, to the extent possible, one or more features of any embodiment can be combined with one or more features of any other embodiment.

## Claims

1. A system (10) configured to manage a sleep session of a subject (12), the system comprising:
an ear insert (14) configured to engage an ear of the subject, the ear insert comprising:
one or more sensory stimulators (16) configured to provide sensory stimulation to the subject during the sleep session; and
one or more sensors (18) configured to generate output signals conveying information related to brain activity of the subject; and
one or more hardware processors (20) configured by machine-readable instructions to:
determine one or more brain activity parameters of the subject based on the output signals; and
control the one or more sensory stimulators based on the one or more brain activity parameters to provide sensory stimulation to the subject to enhance slow wave sleep in the subject during the sleep session.

2. The system of claim 1, wherein:
the one or more physical computer processors are included in a smartphone associated with the subject, the smartphone comprising an input device configured to receive the information in the output signals generated by the one or more sensors;
the one or more sensors comprise one or more electroencephalogram (EEG) electrodes configured to generate EEG output signals that convey the information related to the brain activity of the subject; and
the system further comprises converter and transmitter devices (30) configured convert the EEG output signals for transmission to and receipt by the smartphone input device.

3. The system of claim 2, wherein the one or more EEG electrodes comprise a reference electrode and a sensing electrode.

4. The system of claim 1, wherein the ear insert is custom formed for the subject based on three dimensional data representative of the ear of the subject.

5. The system of claim 1, wherein the one or more sensory stimulators provide audio stimulation to the subject during the sleep session

6. A method for managing a sleep session of a subject (12) with a sleep management system (10), the sleep management system comprising one or more hardware processors (20) and an ear insert (14) that includes one or more sensory stimulators (16) and one or more sensors (18), method comprising:
engaging, with the ear insert, an ear of the subject;
providing, with the one or more sensory stimulators, sensory stimulation to the subject during the sleep session;
generating, with the one or more sensors, output signals conveying information related to brain activity of the subject;
determining, with the one or more hardware processors, one or more brain activity parameters of the subject based on the output signals; and
controlling, with the one or more hardware processors, the one or more sensory stimulators based on the one or more brain activity parameters to provide sensory stimulation to the subject to enhance slow wave sleep in the subject during the sleep session.

7. The method of claim 6, wherein:
the one or more physical computer processors are included in a smartphone associated with the subject, the smartphone comprising an input device configured to receive the information in the output signals generated by the one or more sensors;
the one or more sensors comprise one or more electroencephalogram (EEG) electrodes configured to generate EEG output signals that convey the information related to the brain activity of the subject; and
the system comprises converter and transmitter devices (30) configured convert the EEG output signals for transmission to and receipt by the smartphone input device.

8. The method of claim 7, wherein the one or more EEG electrodes comprise a reference electrode and a sensing electrode.

9. The method of claim 6, further comprising custom forming the ear insert for the subject based on three dimensional data representative of the ear of the subject.

10. The method of claim 6, further comprising providing, with the one or more sensory stimulators, audio stimulation to the subject during the sleep session

11. A system (10) for managing a sleep session of a subject (12), the system comprising:
means (14) for engaging an ear of the subject; the means for engaging comprising:
means (16) for providing sensory stimulation to the subject during the sleep session; and
means (18) for generating output signals conveying information related to brain activity of the subject;
means (20) for determining one or more brain activity parameters of the subject based on the output signals; and
means (20) for controlling the one or more sensory stimulators based on the one or more brain activity parameters to provide sensory stimulation to the subject to enhance slow wave sleep in the subject during the sleep session.

12. The system of claim 11, wherein:
the means for determining and controlling are included in a smartphone associated with the subject, the smartphone comprising means for receiving the information in the output signals generated by the means for generating;
the means for generating comprise one or more electroencephalogram (EEG) electrodes configured to generate EEG output signals that convey the information related to the brain activity of the subject; and
the system further comprises means (30) for converting the EEG output signals for transmission to and receipt by the smartphone means for receiving.

13. The system of claim 12, wherein the one or more EEG electrodes comprise a reference electrode and a sensing electrode.

14. The system of claim 11, further comprising custom forming the means for engaging for the subject based on three dimensional data representative of the ear of the subject.

15. The system of claim 11, further comprising providing, with the means for providing, audio stimulation to the subject during the sleep session
